# EUROPEAN PATENT APPLICATION

(11) **EP 4 575 493 A1**
(43) Date of publication of application: **25.06.2025**
(21) Application number: 23217434.2
(22) Date of filing: 18.12.2023
(51) Int. Cl.: G01N 33/487

(54) **AN ANALOG EVENT DETECTION CIRCUIT FOR A NANOPORE ARRAY**

(71) Applicant: Imec VZW, 3001 Leuven (BE); Katholieke Universiteit Leuven, 3000 Leuven (BE)
(72) Inventor: Van Helleputte, Nick, 3060 Korbeek-Dijle (BE); Marion, Sanjin, 3000 Leuven (BE); Van Dorpe, Pol, 3510 Spalbeek (BE); Sijbers, Wim, 3010 Kessel-lo (BE); Das, Auro, 3000 Leuven (BE)
(74) Representative: Roth, Sebastian

(57) **Abstract**

This disclosure relates to nanopores and nanopore arrays. A readout device for a nanopore array is provided, and an analog detection circuit for the readout device. The detection circuit is connectable to a readout electrode of the nanopore array and, in a detection mode, applies a bias voltage to the readout electrode. Further, it extracts a baseline current from an input current flowing through the readout electrode when the bias voltage is applied, and outputs a digital signal indicating that a current event occurred, if the input current changes by a predetermined threshold value compared to the baseline current. The readout device comprises the detection circuit and a logic device. The logic device determines, based on the digital signal received from the detection circuit, whether a translocation event occurred in the nanopore. In this case, it provides an instruction signal to the detection circuit, to switch from the detection mode to a readout mode

## Description

### TECHNICAL FIELD

The present disclosure relates to nanopores and nanopore arrays. Particularly, the present disclosure provides a readout device for a nanopore array, and an analog detection circuit for the readout device. The readout device, which includes the detection circuit, can detect translocation events in the nanopore array.

### BACKGROUND

Nanopores are a promising technology for next generation sequencing and/or proteomics. For example, solid-state nanopores are a way of fabricating nanopore devices on solid-state (or similar) substrates using lithography. In order to electrically record a signal from such a solid-state nanopore, a constant voltage bias may be applied across the nanopore. This creates a current flow through the nanopore, and when a (charged) molecule passes through the nanopore, this current is modulated. An electrical current amplifier can record the modulated current, and the molecule that passed through the nanopore can be identified based thereon. This approach is called current-based sensing.

Translocation events happen according to stochastic processes. Nanopores cannot be continuously filled with molecules, and typical nanopore signals consequently are fairly sparse. This means, that any single nanopore signal will contain a large portion of (mostly) irrelevant baseline current, while the actual translocation event signal is only a very small part of the overall recorded signal. However, since a translocation event can occur at any point in time, the nanopore signal is typically recorded continuously, and the useful parts of the signal are then identified offline in post-processing.

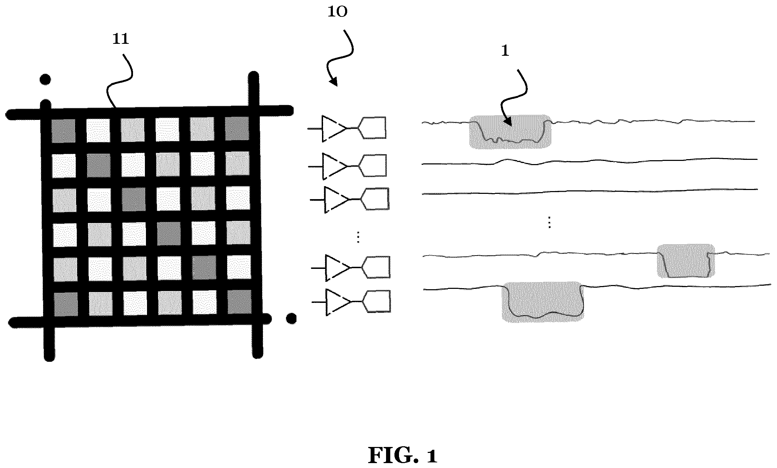

FIG. 1 shows a conceptual view of a nanopore array 11 and a conventional readout device 10. The nanopore array 11 comprises a plurality of nanopores with corresponding fluidic channels (not shown). Each nanopore has its own readout chain in the readout device 10, wherein the readout chain typically includes a transimpedance amplifier and a high-speed analog-to-digital converter. The raw digital output signal stream goes to a signal processing block, which will identify translocation events in the signal stream, which will then be further analyzed (for example, using basecalling algorithms).

Since the raw signal streams of the individual nanopores are sparse, having only small portions ("current events 1", illustrated by the dips in the exemplary signal shown in FIG. 1) which may pertain to actual translocation events, and since many readout chains and many analog-to-digital converters are needed, significant silicon area and power are required, although most of these circuit building blocks will process irrelevant data most of the time. This creates a data bottleneck especially for large nanopore arrays. For example, a nanopore array of 50.000 nanopores, each read at 1 Msps with a typical resolution of 10 bits, will generate a raw signal stream of 500 Gbps. However, typical translocation rates can be anywhere from a few % to a few tens of percent at best, meaning that from this 500 Gbps, only a very small fraction contains actual data related to translocation events.

### SUMMARY

In view of the above, the present disclosure aims to provide an improved readout solution for nanopore array. An objective is to reduce the amount of data to be processed, and thus to remove the mentioned data bottleneck. An ideal objective is to process only data relating to translocation events, and no irrelevant data.

These and other objectives are achieved by the solutions of the present disclosure, as described in the independent claims. Advantageous implementations are described in the dependent claims.

A first aspect of this disclosure provides a detection circuit for detecting translocation events in a nanopore array, wherein the detection circuit is connectable to a readout electrode of a nanopore of the nanopore array, and is configured to, in a detection mode: apply a bias voltage to the readout electrode; extract a baseline current from an input current, which flows through the readout electrode into the detection circuit when the bias voltage is applied; and output a digital signal indicating that a current event occurred, if the input current changes by at least a predetermined threshold value compared to the baseline current.

The detection circuit of the first aspect is able to indicate that the input current deviates from the baseline current. This makes it possible to process the data provided by the nanopore only in case of a current event, which may correspond to a translocation event. In this respect, in an exemplary approach of this disclosure, a current event can be used as equivalent of a translocation event, i.e., any current event can be treated as a translocation event. In another approach, current events could be further analyzed for "false positives", in order to determine translocation events. As a result of using the detection circuit, only translocation events may be processed, but no irrelevant data has to be processed. The detection circuit of the first aspect therefore enables a reduction of the amount of data to be processed, and thus removes the mentioned data bottleneck.

In an implementation of the detection circuit, the detection circuit is further connectable to a logic device and to a readout chain, respectively, and is further configured to, in the detection mode: receive a first instruction signal from the logic device; switch from the detection mode to a readout mode in response to the first instruction signal; and in the readout mode, provide the input current directly to the readout chain.

The readout chain thus receives data in case of the readout mode. In the readout mode, the detection circuitry of the detection circuit may be disconnected, i.e., the detection circuit would not perform the baseline current extraction and current comparison, which it is configured to perform in the detection mode. In the detection mode, the readout chain may be disconnected from the nanopore, i.e., only detection of current events but no readout is performed with the detection circuit. Accordingly, the detection circuit can be triggered to enable the readout of the nanopore only in response to the detected current event. This reduces the amount of read data, which has to be processed.

In an implementation of the detection circuit, the detection circuit is further configured to, in the readout mode: receive a second instruction signal from the logic device; and switch from the readout mode to the detection mode in response to the second instruction signal.

In an implementation of the detection circuit, the detection circuit comprises: a biasing circuit configured to apply the bias voltage to the readout electrode and to receive the input current; a current mirroring circuit connected to the biasing circuit, wherein the current mirroring circuit comprises a low-pass filter configured to extract the baseline current, and is configured to provide a difference between the extracted baseline current and the input current to a current comparator circuit; and the current comparator circuit configured to compare the difference between the baseline current and the input current with the predetermine threshold value; wherein the detection circuit is configured to output the digital signal indicating that a current event occurred, if the difference between the baseline current and the input current exceeds the predetermined threshold value.

The mirroring circuit may thus have two paths. One path for providing the input current through the low-pass filter, so as to extract the baseline current, and another path for the real-time input current. The output of the mirroring circuit is in this case the difference between the real time current signal and the baseline current. The current comparator compares this difference current with the threshold current, and the current event is `flagged', when this difference current exceeds the threshold.

In an implementation of the detection circuit, the detection circuit further comprises: a first switch configured to disconnect the biasing circuit from the readout electrode in response to receiving the first instruction signal, and to connect the biasing circuit to the readout electrode in response to receiving the second instruction signal; and a second switch configured to connect the readout electrode directly to the readout chain in response to receiving the first instruction signal, and to disconnect the readout electrode from the readout chain in response to receiving the second instruction signal.

The first and second switch are a simple and fast way to operate the detection circuit based on the digital output signal, i.e., based on whether or not a current event is detected.

A second aspect of this disclosure provides a readout device for a nanopore array, wherein the readout device comprises: a plurality of detection circuits according to the first aspect and any implementation thereof, wherein each detection circuit is connectable to a respective readout electrode of one of a plurality of nanopores of the nanopore array; and a logic device connected to the detection circuits; wherein the logic device is configured to: receive, from a respective detection circuit of the detection circuits, a digital signal indicating that one or multiple current events occurred; determine, based on the digital signal, whether a translocation event occurred in the respective nanopore to which the respective detection circuit is connected; and provide a first instruction signal to the respective detection circuit, to switch the respective detection circuit from the detection mode to the readout mode, if determining that a translocation event occurred.

The readout device is able to identify translocation events in the current signal from the nanopore based on the digital signal received from the detection circuit, and is configured to connect the nanopore only to the readout chain, if it identifiers a translocation event. Consequently, only translocation event related data of the nanopore may be processed in the readout chain or after, but no irrelevant data. The readout device of the second aspect therefore enables a reduction of the amount of data to be processed, and thus removes the mentioned data bottleneck.

In an implementation of the readout device, the logic device is further configured to provide a second instruction signal to the respective detection circuit, to switch the respective detection circuit back to the detection mode.

In an implementation of the readout device, the logic device is configured to provide the second instruction signal after a predetermined period of time has passed from providing the first instruction signal, or in response to a second digital signal received from the respective detection circuit, the second digital signal indicating that the one or multiple current events are over.

This may help to ensure that no translocation event is missed.

In an implementation of the readout device, the logic device is configured to always determine that a translocation event occurred if a current event has occurred.

This is a straightforward implementation, and may ensure that no translocation event is missed.

In an implementation of the readout device, the logic device is configured to determine that a translocation event occurred, if the multiple current events exhibit a predetermined pattern of occurrence.

In an implementation of the readout device, wherein a translocation event is related to a particularly patterned molecule passing through the nanopore, and the logic device is configured to determine that a translocation event occurred, if the one or multiple current events resemble predetermined one or multiple current events associated with the particularly patterned molecule.

This more sophisticated implementation may help to avoid the processing of "false positives", i.e., current events which are detected but do not correspond to real translocation events. Further, this implementation may also allow distinguishing between translocation events of interest (e.g., attributed to a specific type of molecules) and irrelevant translocation events (e.g., attributed to other types of molecules, which may also pass through the nanopore, but which are not of interest).

In an implementation of the readout device, the readout device further comprises one or more readout chains connected to the detection circuits, and configured to readout all the nanopores of the nanopore array, which are connected to a detection circuit in the readout mode.

In an implementation of the readout device, a respective group of the detection circuits is multiplexed into each readout chain.

This implementation may reduce the area required for the readout chain of the readout device, and may thus help in scaling down the readout device.

In an implementation of the readout device, the readout device is configured to provide, within a certain period of time, a first instruction signal to only one of the detection circuits of the group.

This may help to avoid a mix-up in the readout chain of translocation events stemming from different nanopores.

A third aspect of this disclosure provides a detection method for detecting translocation events in a nanopore array, wherein the detection method comprises: applying a bias voltage to a readout electrode of a nanopore of the nanopore array; extracting a baseline current from an input current, which flows through the readout electrode when the bias voltage is applied; determining that a current event occurred, if the input current changes by at least a predetermined threshold value compared to the baseline current; determining, based on the current event, whether a translocation event occurred in the nanopore; and providing the input current directly to a readout chain, if determining that a translocation event occurred.

The method of the third aspect achieves the same advantages as the readout device of the second aspect, and may be extended by respective implementations as described above for the detection circuit of the first aspect and the readout device of the second aspect, respectively.

In summary, the solutions of this disclosure are based on the proposal of a novel detection circuit, which can detect current events in the current signal of a nanopore (which may relate to translocation events), particularly, directly at the nanopore side and before any analog signal amplification or analog-to-digital conversion. Only nanopores that have current events will in this way be connected to a readout chain, for instance, including an amplifier and analog-to-digital converter. As a consequence, ideally, only data related to translocation events is processed and sent off-chip, which significantly increases the achievable useful data throughput on large-scale nanopore arrays.

Moreover, the detection circuit of this disclosure supports a multiplexing of multiple nanopores into a single readout chain. Since it is unlikely that neighboring nanopores will have translocation events at the same time, there may be no need to physically provide a complete signal amplification and analog to-digital conversion chain for each nanopore in the nanopore array. This means that less silicon area may be needed for the readout device in a large nanopore array, which further enhances the scalability.

### BRIEF DESCRIPTION OF THE DRAWINGS

The above described aspects and implementations are explained in the following description of embodiments with respect to the enclosed drawings:
- FIG. 1: shows a conventional readout device for a nanopore array.
- FIG. 2: shows a detection circuit according to this disclosure for a nanopore array.
- FIG. 3: shows a readout device according to this disclosure for a nanopore array.
- FIG. 4: shows another readout device according to this disclosure for a nanopore array.
- FIG. 5: shows an exemplary detection circuit according to this disclosure for a nanopore array.
- FIG. 6: shows details of an exemplary detection circuit according to this disclosure for a nanopore array.
- FIG. 7: shows a flow diagram of a method according to this disclosure for detecting translocation events in a nanopore array.

### DETAILED DESCRIPTION OF EMBODIMENTS

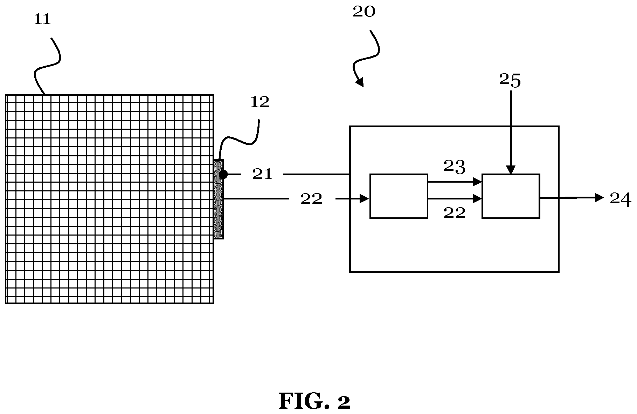

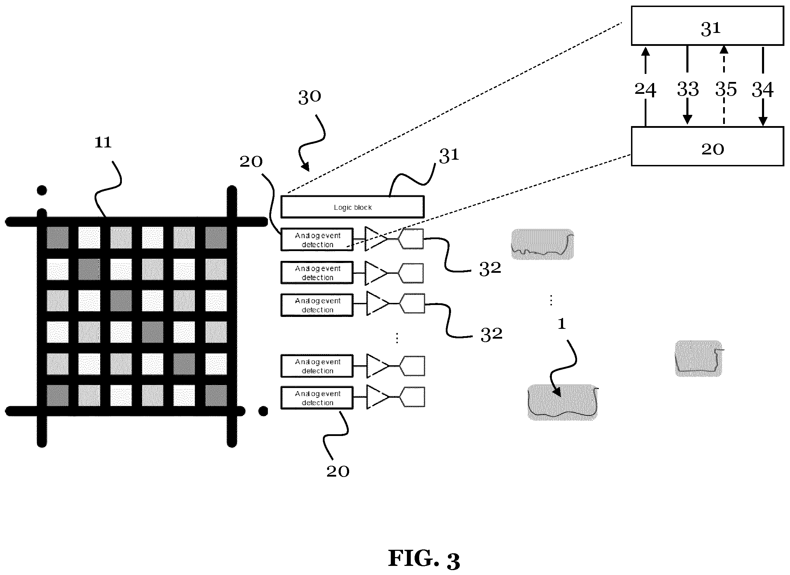

FIG. 2 shows a detection circuit 20 according to this disclosure, which maybe used for or with a nanopore array 11. The detection circuit 20 may particularly be used to detect current events 1 in the nanopore array 11, which may related to translocation events in the nanopore array 11. Thus, the detection circuit 20 may be used to detect translocation events in the nanopore array 11. For instance, the detection circuit 20 may be part of a readout device 30 (see FIG. 3), which can be used to readout the nanopore array 11, and particularly may be able to determine translocation events in the nanopore array 11. To use the detection circuit 20 in the before-mentioned way, it is connectable to a readout electrode 12 of a nanopore of the nanopore array 11. The readout electrode 12 of the nanopore array 11 may be a "Trans" electrode, but may also be a "Cis" electrode (notably, it is not drawn to scale in FIG. 2). The nanopore array 11 may comprise solid-state nanopores, for example, but any conventional nanopore array may be used, as long as it comprises readout electrodes and allows current-based sensing.

The detection circuit 20 may comprise circuitry (not shown in FIG. 2), which is configured to perform, conduct or initiate the various operations of the detection circuit 20 described in the following. The circuitry may comprise hardware and/or may be controlled by software. The hardware may comprise analog circuitry or digital circuitry, or both analog and digital circuitry. The analog circuitry may comprise transistors, diodes, amplifiers, capacitors, filters, or the like. The digital circuitry may comprise components such as application-specific integrated circuits (ASICs), field-programmable arrays (FPGAs), digital signal processors (DSPs), or multi-purpose processors.

In particular, the detection circuit 20 may be configured to operate in a detection mode, in which it is configured to apply a bias voltage 21 to the readout electrode 12, and to extract a baseline current 23 from an input current 22, which flows through the readout electrode 12 into the detection circuit 20 when the bias voltage 21 is applied. Further, the detection circuit 20 is configured to, in the detection mode, output a digital signal 24 indicating that a current event 1 occurred, if the input current 22 changes by at least a predetermined threshold value 25 compared to the baseline current 23. The digital signal 24 may be a flag, which indicates the current event 1. The detection circuit 20 may also operate in a readout mode, as explained below.

FIG. 3 shows a readout device 30 according to this disclosure, which is configured to readout the nanopore array 11. The readout device 30 may comprise one or more detection circuits 20 as the one described with respect to FIG. 1. For instance, the readout device 30 may comprise a plurality of such detection circuits 20. In this case, each detection circuit 20 maybe connectable to a respective readout electrode 12 of one of a plurality of nanopores of the nanopore array 11, i.e., each detection circuit 20 may be associated with one of the nanopores of the nanopore array 11.

The readout device 30 may comprise a logic device 31, which is connected to the one or more detection circuits 20. The logic device 30 may be a processor or a controller. The logic device 30 may comprise multiple logic units, for instance, sub-controllers or sub-processors, wherein one logic unit may be associated with each respective detection circuit 20.

The logic device 30 may comprise processing circuitry (e.g., one or more processors, not shown) configured to perform, conduct or initiate the operations of the logic device 30 described in the following. The processing circuitry may comprise hardware and/or the processing circuitry may be controlled by software. The hardware may comprise analog circuitry or digital circuitry, or both analog and digital circuitry. The digital circuitry may comprise components such as application-specific integrated circuits (ASICs), field-programmable arrays (FPGAs), digital signal processors (DSPs), or multi-purpose processors. The logic device 30 may further comprise memory circuitry, which stores one or more instruction(s) that can be executed by the processing circuitry, in particular under control of the software. For instance, the memory circuitry may comprise a non-transitory storage medium storing executable software code which, when executed by the processing circuitry, causes the various operations of the logic device to be performed.

In particular, the logic device 31 is configured to (see the zoom-in in FIG. 3) receive, from a respective detection circuit 20 of the one or more detection circuits 20, a digital signal 24, which indicates that one or multiple current events 1 occurred. The logic device 31 is then configured to determine, based on the digital signal 24, whether a translocation event occurred in the respective nanopore, to which the respective detection circuit 20 is connected. For example, the logic device 31 may be configured to always determine that a translocation event occurred, if a current event 1 has occurred. In this case, a current event 1 detected by the detection circuit 20 corresponds to a translocation event identified by the logic device 31. The logic device 31 may simply evaluate the digital signal 24, which may correspond to a flag, for the presence of a current event 1. However, it may also be possible to configure the logic device 31 in a more complex way, for instance, to determine that a translocation event occurred only, if multiple current events 1 occurred according to a predetermined pattern of occurrence. For instance, if multiple current events 1 occurred within a certain period of time, or if multiple current events 1 follow some predetermined event pattern.

If the logic device 31 determines that a translocation event occurred, it is configured to provide a first instruction signal 33 to the respective detection circuit 20. The first instruction signal 33 instructs the respective detection circuit 20 to switch from its detection mode to its readout mode. Accordingly, each detection circuit 20 of the readout device 30 may be configured to switch from the detection mode to the readout mode, if it receives a first instruction signal 33 from the logic device 31.

As shown in FIG. 3, the readout device 30 further comprises one or more readout chains 32 connected to the one or more detection circuits 20. Each readout chain 32 may be associated with one detection circuit 20, as particularly shown in FIG. 3. In the readout mode, or when a respective detection circuit 20 switches from the detection mode to the readout mode, the respective detection circuit 20 is configured to provide the input current 22 directly to the readout chain 32. In the detection mode, the respective detection circuit 20 may be disconnected from the associated readout chain 32. The readout chains 32 are thus configured to readout (only) all the nanopores of the nanopore array 11, which are connected to a detection circuit 20 that is in the readout mode.

The logic device 31 is further configured to provide a second instruction signal 34 to the respective detection circuit 20, which instructs the respective detection circuit 20 to switch (back) to the detection mode. Accordingly, each detection circuit 20 of the readout device 30 may be configured to switch from the readout mode to the detection mode, if it receives a second instruction signal 34 from the logic device 31. The logic device 31 may be configured to provide the second instruction signal 34 to the respective detection circuit 20 after a predetermined period of time has passed from providing the first instruction signal 33 to the respective detection circuit 20. Alternatively, the logic device 31 could receive a second optional digital signal 35 from the respective detection circuit 20, which could indicate that the previously indicated current event 1 is over. In response to receiving this further digital signal 35, the logic device 31 may send the second instruction signal 34 to the respective detection circuit 20.

In summary of the above, the present disclosure proposes an analog event detection circuit 20. This detection circuit 20 may be a rather small circuit, which is able to detect when a significant current deviation from the baseline current 23 of a nanopore of the nanopore array 11 happens. This is recognized as a current event 1 and may be identified as a translocation event by the logic device 31. Only in case of a translocation event, the respective nanopore of the nanopore array 11 may be connected to a signal amplification and analog-to-digital conversion channel. The solution of this disclosure allows significantly reducing the raw signal stream, since only data following a rapid change in baseline current 23 may have to be converted and further analyzed, for instance, in order determine if it was a true translocation event, and if so, for instance, to perform a suitable analysis (e.g., basecalling or barcode detection).

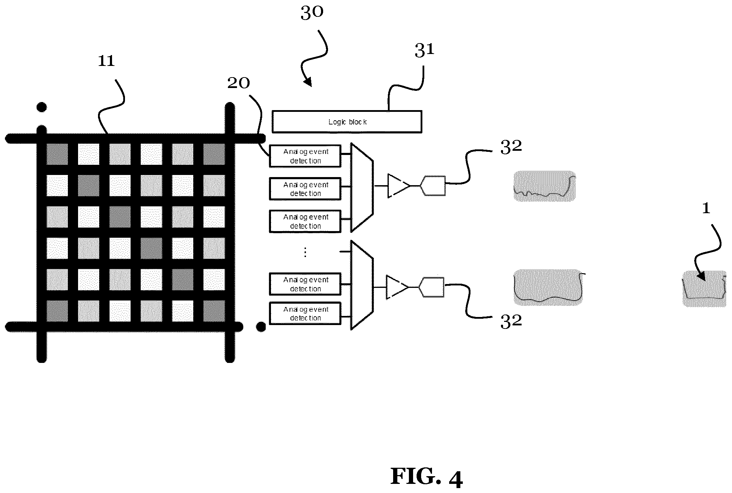

FIG. 4 shows another readout device 30 according to this disclosure, which is similar to the readout device 30 shown in FIG. 3. Thus, same elements in FIG. 3 and FIG. 4 are labelled with the same reference signs and may be implemented likewise. Only the differences between FIG. 3 and FIG. 4 are described in the following.

As shown, a respective group of the detection circuits 20 is multiplexed into each readout chain 32 in the readout device 30 of FIG. 4. That means, each readout chain 32 may be associated with more than one detection circuit 20, unlike in the readout device 30 of FIG. 3. In this alternative implementation, multiple nanopores, each with their own detection circuit 20, can be multiplexed into a single readout chain 32. This may be useful to further reduce the amount of hardware needed. The number of nanopores, which can be multiplexed into a single readout chain 32, may depend on the expected translocation rate. As a feasible example, the number of multiplexed nanopores may be in a range of 5-50 nanopores. Of course, also two, three, or four nanopores may be multiplexed into one readout chain 32.

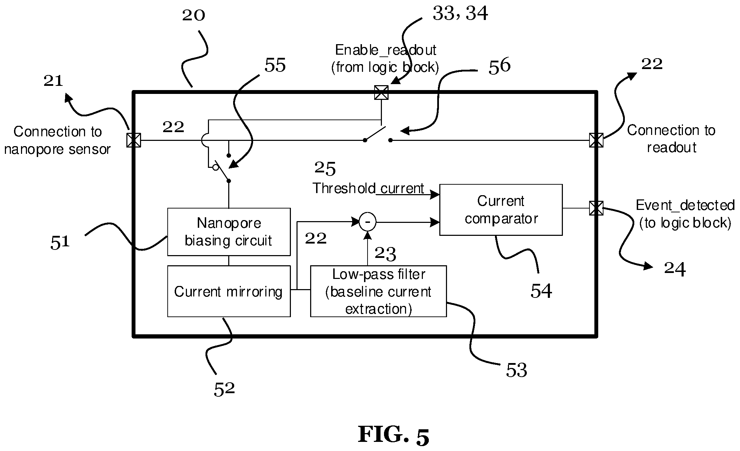

FIG. 5 shows an exemplary detection circuit 20 according to this disclosure, which may implement the detection circuits 20 shown in the previous figures. Same elements in FIG. 5 and the previous figures are labelled with the same reference signs and may be implemented likewise.

The detection circuit 20 of FIG. 5 comprises a biasing circuit 51, which is configured to apply the bias voltage 21 to the readout electrode 12, and to receive the input current 22 that flows through the readout electrode 12 into the detection circuit 20 when the bias voltage is applied. The detection circuit 20 comprises a first connection suitable to connect it to the nanopore array 11 ("Connection to nanopore sensor").

The detection circuit 20 of FIG. 5 further comprises a current mirroring circuit 52, which is connected to the biasing circuit 51. The current mirroring circuit 52 may comprise a low-pass filter 53, which is configured to extract the baseline current 23. The current mirroring circuit 52 is configured to provide a difference between the extracted baseline current 23 and the received input current 22 to a current comparator circuit 54 of the detection circuit 20.

The current comparator circuit 54 is configured to compare the difference between the baseline current 23 and the input current 22 with the predetermined threshold value 25. The predetermined threshold value 25 may be variable, for example, in order to account for different scenarios in which the nanopore array 11 is used. The threshold value 25, which is a threshold current, may be provided to the detection circuit 20.

The detection circuit 20 is then configured to output the digital signal 24, which indicates whether or not a current event occurred, e.g., a flag that is set to "1" in case of a current event, if the difference between the baseline current 23 and the input current 22 exceeds the predetermined threshold current 25. The detection circuit 20 can output the digital signal 24 via a second connection that is suitable to connect it to the logic device ("Event_detected").

The detection circuit 20 of FIG. 5 further comprises a third connection that is also suitable to connect it to the logic device 31 ("Enable_readout"). In particular, the third connection is configured to receive the first or the second instruction signal 33, 34 from the logic device 31. The detection circuit 20 of FIG. 5 further comprises a first switch 55, which is configured to disconnect the biasing circuit 51 from the readout electrode 12 in response to receiving the first instruction signal 33. The first switch 55 is further configured to connect the biasing circuit 51 to the readout electrode 12 in response to receiving the second instruction signal 34. For instance, the first and second instruction signals 33, 34 may have "high" and "low" values, which may open or close the respective switch 55, 56.

The detection circuit 20 further comprises a fourth connection that is suitable to connect it to the readout chain 32 associated with the detection circuit ("Connection to readout"). The second switch 56 is configured to connect the readout electrode 12 directly to the fourth connection to the readout chain 32 in response to receiving the first instruction signal 34. The second switch 56 is further configured to disconnect the readout electrode 12 from the readout chain 32 in response to receiving the second instruction signal 34.

FIG. 5 illustrates a more abstract architecture of the detection circuit 20 and its above-described sub-circuits 51, 52, 53, 54 and switches 55, 56. The detection circuit's operation can be summarized as follows:
1. Switches 55, 56: The nanopore associated with the detection circuit 20 can be either connected to the readout chain 32 (in the "readout mode", the first switch 55 is closed) or to the event detection circuitry 52, 53, 54 (in the "detection mode", the second switch 56 is closed). The switch control is handled by the logic device 31 via the third connection. When the first switch 55 is closed, the analog event detection circuitry 52, 53, 54 is disabled and the nanopore signal can be amplified, digitized and recorded as normal.
2. Nanopore biasing circuit 51: The actual nanopore associated with the detection circuit 20 must be properly biased. More correctly, the recording electrode in the "Trans" chamber must be held at a suitable voltage. This is needed to maintain proper nanopore operation, and to ensure translocation events can occur. In the readout mode of the detection circuit 20, this could be done by the readout chain 32. However, at least in the detection mode (second switch 56 closed, first switch 55 open), the dedicated nanopore biasing circuitry 51 may handle this.
3. Baseline current extraction circuit 53: The baseline current 23 is extracted. Due to process variability, each nanopore may be expected to have slightly different baseline currents 23, and baseline currents 23 can potentially drift over time.
4. Compare comparator circuit 54: Compares the instantaneous nanopore current 22 to an event detection threshold current 25. The actual instantaneous nanopore current 22 may be subtracted from the extracted baseline current 23. When this instantaneous current exceeds a certain threshold 25 below the baseline current 23, the detection circuit 20 may trigger the digital output signal 24. The threshold value 25 is beneficially be chosen large enough so that random nanopore noise will not cause a triggering, but also small enough that normal translocation events will be triggered. The threshold current 25 could be globally set or could also be programmable per nanopore if so desired. A good rule of thumb may be to choose a threshold value 25 that is in a range of 3x-9x larger than the baseline root mean square noise, in order to avoid too many false positive triggers. The threshold value 25 is beneficially between 50% and 70% of normal expected translocation currents, in order to avoid that actual translocation events are missed or detected too late.

The output signal 24 is an indication that a translocation event has likely started, as it indicates a current event 1. The logic device 31 may in this case disconnect the analog event detection circuitry 52, 53, 54, and may connect the nanopore to a signal readout chain 32, so that the translocation signal can be recorded. The end of the recording can be either a fixed timing, if typical translocation event durations can be assumed predictable and reasonably constant, or could be determined by another digital signal processing block (not shown) that detects a return to baseline current 23 in the recorded signal. The logic device 31 can also ignore triggers from certain nanopores. This may happens, if any of the following conditions is met:
1. If multiple nanopores are multiplexed into a single readout chain 32 (as in FIG. 4), if a translocation event happens on a nanopore, that nanopore will be connected to the readout chain 32. If a translocation event happens while the readout chain 32 is already handling another event, the new event will be ignored.
2. Dirty, broken, poorly wetted or otherwise badly behaving nanopores can occur in large scale nanopore arrays 11. It is conceivable that a few nano pores will not be functional as intended. This can be because they are dirty, broken, not properly wet or for a number of other reasons. This might lead to continuous triggers, which would lead to a lot of useless data and might block the readout chain 32. It is possible to ignore such bad nanopores.

The logic device 31 may further also be configured to force a normal recording from any specific nanopore at any specific time by simply turning the connection "Enable_readout" high (first instruction signal 33), independent of the "Event_detected" state, i.e., the output signal 24. This can for example be useful for initial calibration (i.e. recording baseline currents) or to perform standard quality control checks.

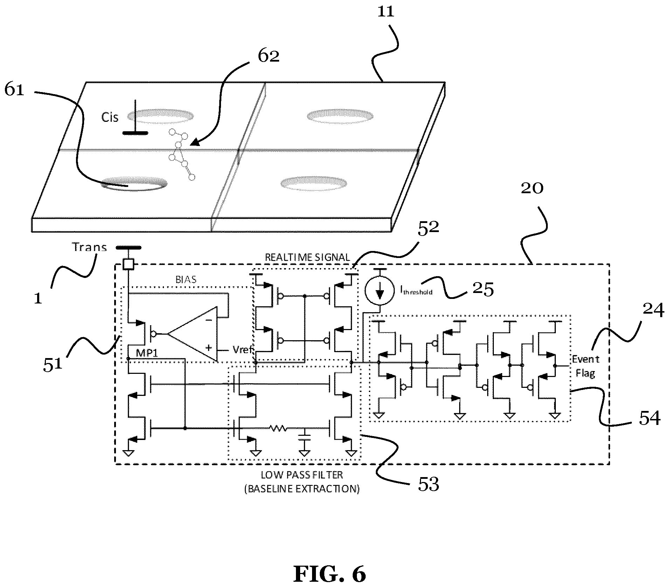

FIG. 6 shows, in contrast to FIG. 5, a more detailed architecture of an exemplary detection circuit 20 according to this disclosure for a nanopore array 11 having nanopores 61, and may implement the detection circuit 20 of the previous figures. A loop formed by an amplifier and a common-gate stage MP1 may set the bias on the readout electrode 12 (the detection circuit 20 is exemplarily connected to the "Trans" electrode here) to the voltage Vref, i.e., may function as the biasing circuit 51. The input current signal 22 from the readout electrode 12 is then copied through the current mirror to the two blocks referred to as `REALTIME SIGNAL' and `LOW PASS FILTER'. The low-pass filter 53 is implemented with a resistor and a capacitor, and is used to extract the baseline current 23 of the input current 22 received from the readout electrode 12. This is subtracted from the real time signal copied in the upper block. This difference is propagated to the next stage. A threshold current 25 is added at this node, such that the difference current is compared with the threshold current by the current comparator circuit 54. When the difference current changes to below the threshold current value 25, the current comparator circuit 54 sends an event flag as the digital signal 24 to the logic device 31. This may occur in case of a translocation event, i.e., a molecule 62 passing through the nanopore 61. The flag may be reset when the input current 22 returns to the baseline current 23.

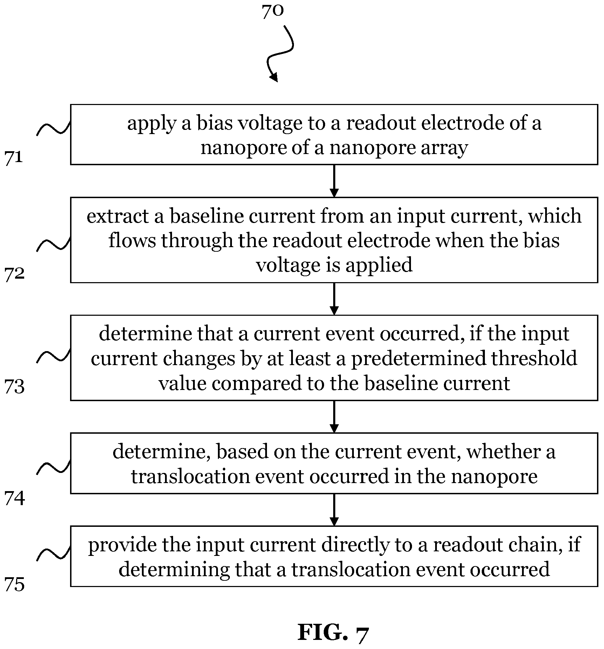

FIG. 7 shows a flow diagram of a method 70 for detecting translocation events in a nanopore array 11, according to this disclosure. The method 70 may be performed by a readout device 30 to detect translocation events. The method 70 comprises a step 71 of applying a bias voltage 21 to a readout electrode 12 of a nanopore of the nanopore array 11, and a step 72 of extracting a baseline current 23 from an input current 22, which flows through the readout electrode 12 when the bias voltage 21 is applied. The method 70 further comprises a step 73 of determining that a current event 1 occurred, if the input current 22 changes by at least a predetermined threshold value 25 compared to the baseline current 23. The method 70 then comprises a step 74 of determining, based on the current event 1, whether a translocation event occurred in the nanopore, and a step 75 of providing the input current 22 directly to a readout chain 32, if determining that a translocation event occurred.

In conclusion, this disclosure proposes an analog event detection circuit 20 that is able to identify potential translocation events through a nanopore of a nanopore array 11 prior to any signal amplification and digitization. The output of this analog detection circuit 20 may allow only amplifying and digitizing data pertaining to actual translocation events ultimately resulting in much less data that must be recorded and processed.

In the claims as well as in the description of this disclosure, the word "comprising" does not exclude other elements or steps and the indefinite article "a" or "an" does not exclude a plurality. A single element may fulfill the functions of several entities or items recited in the claims. The mere fact that certain measures are recited in the mutual different dependent claims does not indicate that a combination of these measures cannot be used in an advantageous implementation.

## Claims

1. A detection circuit (20) for detecting translocation events in a nanopore array (11), wherein the detection circuit (20) is connectable to a readout electrode (12) of a nanopore (61) of the nanopore array (11), and is configured to, in a detection mode:
apply a bias voltage (21) to the readout electrode (12);
extract a baseline current (23) from an input current (22), which flows through the readout electrode (12) into the detection circuit (20) when the bias voltage (21) is applied; and
output a digital signal (24) indicating that a current event (1) occurred, if the input current (22) changes by at least a predetermined threshold value (25) compared to the baseline current (23).

2. The detection circuit (20) according to claim 1, wherein the detection circuit (20) is further connectable to a logic device (31) and to a readout chain (32), respectively, and is further configured to, in the detection mode:
receive a first instruction signal (33) from the logic device (31);
switch from the detection mode to a readout mode in response to the first instruction signal (33); and
in the readout mode, provide the input current (22) directly to the readout chain (32).

3. The detection circuit (20) according to claim 2, further configured to, in the readout mode:
receive a second instruction signal (34) from the logic device (31); and
switch from the readout mode to the detection mode in response to the second instruction signal (34).

4. The detection circuit (20) according to one of the claims 1 to 3, comprising:
a biasing circuit (51) configured to apply the bias voltage (21) to the readout electrode (12) and to receive the input current (22);
a current mirroring circuit (52) connected to the biasing circuit (51), wherein the current mirroring circuit (52) comprises a low-pass filter (53) configured to extract the baseline current (23), and is configured to provide a difference between the extracted baseline current (23) and the input current (22) to a current comparator circuit (54); and
the current comparator circuit (54) configured to compare the difference between the baseline current (23) and the input current (22) with the predetermined threshold value (25);
wherein the detection circuit (20) is configured to output the digital signal (24) indicating that a current event (1) occurred, if the difference between the baseline current (23) and the input current (22) exceeds the predetermined threshold value (25).

5. The detection circuit (20) according to the claims 2, 3 and 4, further comprising:
a first switch (55) configured to disconnect the biasing circuit (51) from the readout electrode (12) in response to receiving the first instruction signal (33), and to connect the biasing circuit (51) to the readout electrode (12) in response to receiving the second instruction signal (34); and
a second switch (56) configured to connect the readout electrode (12) directly to the readout chain (32) in response to receiving the first instruction signal (33), and to disconnect the readout electrode (12) from the readout chain (32) in response to receiving the second instruction signal (34).

6. A readout device (30) for a nanopore array (11), wherein the readout device (30) comprises:
a plurality of detection circuits (20) according to one of the claims 1 to 5, wherein each detection circuit (20) is connectable to a respective readout electrode (12) of one of a plurality of nanopores (61) of the nanopore array (11); and
a logic device (31) connected to the detection circuits (20);
wherein the logic device (31) is configured to:
receive, from a respective detection circuit (20) of the detection circuits (20), a digital signal (24) indicating that one or multiple current events (1) occurred;
determine, based on the digital signal (24), whether a translocation event occurred in the respective nanopore (61) to which the respective detection circuit (20) is connected; and
provide a first instruction signal (33) to the respective detection circuit (20), to switch the respective detection circuit (20) from the detection mode to the readout mode, if determining that a translocation event occurred.

7. The readout device (30) according to claim 6, wherein the logic device (31) is further configured to provide a second instruction signal (34) to the respective detection circuit (20), to switch the respective detection circuit (20) back to the detection mode.

8. The readout device (30) according to claim 7, wherein the logic device (31) is configured to provide the second instruction signal (34) after a predetermined period of time has passed from providing the first instruction signal (33), or in response to a second digital signal (35) received from the respective detection circuit (20), the second digital signal (35) indicating that the one or multiple current events (1) are over.

9. The readout device (30) according to one of the claims 6 to 8, wherein the logic device (31) is configured to always determine that a translocation event occurred if a current event (1) has occurred.

10. The readout device (30) according to one of the claims 6 to 8, wherein the logic device (31) is configured to determine that a translocation event occurred, if the multiple current events (1) exhibit a predetermined pattern of occurrence.

11. The readout device (30) according to one of the claims 6 to 8, wherein a translocation event is related to a particularly patterned molecule (62) passing through the nanopore, and the logic device (31) is configured to determine that a translocation event occurred, if the one or multiple current events (1) resemble predetermined one or multiple current events (1) associated with the particularly patterned molecule (62).

12. The readout device (30) according to one of the claims 1 to 11, further comprising one or more readout chains (32) connected to the detection circuits (20), and configured to readout all the nanopores (61) of the nanopore array (11), which are connected to a detection circuit (20) in the readout mode.

13. The readout device (30) of claim 12, wherein a respective group of the detection circuits (20) is multiplexed into each readout chain (32).

14. The readout device (30) of claim 13, wherein the readout device (30) is configured to provide, within a certain period of time, a first instruction signal (33) to only one of the detection circuits (20) of the group.

15. A detection method (70) for detecting translocation events in a nanopore array (11), wherein the detection method (70) comprises:
applying (71) a bias voltage (21) to a readout electrode (12) of a nanopore (61) of the nanopore array (11);
extracting (72) a baseline current (23) from an input current (22), which flows through the readout electrode (12) when the bias voltage (21) is applied;
determining (73) that a current event occurred, if the input current (22) changes by at least a predetermined threshold value (25) compared to the baseline current (23);
determining (74), based on the current event (1), whether a translocation event occurred in the nanopore (61); and
providing (75) the input current (22) directly to a readout chain (32), if determining that a translocation event occurred.
